# EUROPÄISCHE PATENTSCHRIFT

(11) **EP 3 677 242 B1**
(45) Veröffentlichungstag und Bekanntmachung des Hinweises auf die Patenterteilung: **03.04.2024**
(21) Anmeldenummer: 19217912.5
(22) Anmeldetag: 19.12.2019
(51) Int. Cl.: A61K 8/00, A61K 8/02, A61K 8/04, A61K 8/19, A61K 8/27, A61K 8/29, A61K 8/34, A61Q 17/00, A61Q 19/10, A01N 25/04, A01N 31/02, A01P 1/00

(54) **VERWENDUNG EINER DESINFEKTIONSZUSAMMENSETZUNG ZUR HAUTDESINFEKTION**
USE OF A DISINFECTANT COMPOSITION FOR DISINFECTING THE SKIN
UTILISATION D'UNE COMPOSITION DÉSINFECTANTE POUR DÉSINFECTER LA PEAU

(30) Priorität: 20.12.2018 DE 102018133179
(43) Veröffentlichungstag der Anmeldung: 08.07.2020
(73) Patentinhaber: Brauns-Heitmann GmbH & Co. KG, 34414 Warburg (DE)
(72) Erfinder: Gibbels, Dr. Uwe, 34414 Warburg (DE); Nentwig, Rüdiger, 34431 Marsberg (DE)
(74) Vertreter: Geskes, Christoph

(56) Entgegenhaltungen:
- WO-A1-99/59540
- WO-A1-2017/144834
- DD-A3- 206 878
- DE-T2- 69 920 538
- JP-A- H1 135 403
- US-A1- 2005 271 595
- Floratech: "Florasomes and Floraspheres", , Juni 2018 (2018-06), XP055699589, Gefunden im Internet: URL:https://www.floratech.com/PDFs/PIBs/PI B_Floraspheres.pdf [gefunden am 2020-05-29]

## Beschreibung

Die vorliegende Erfindung betrifft eine nicht-therapeutische Verwendung einer Desinfektionszusammensetzung zur Hautdesinfektion in der Form eines Gels, umfassend mindestens einen Alkohol, Wasser und mindestens ein Verdickungsmittel.

Aus dem Stand der Technik sind Desinfektionszusammensetzungen insbesondere für Hände bekannt. Desinfektionszusammensetzungen werden im Alltag benutzt für die Desinfektion und die Reinigung von Händen und sonstigen Körperteilen. Es ist bekannt, dass Desinfektionsmittel Alkohol und Wasser umfassen. Alkohol ist für die Desinfektion geeignet. Alkohol ist bakterizid, levurozid, fungizid und teilweise viruzid.

US 2005/271595 A1 offenbart eine desinfizierende Zusammensetzung in Form einer viskosen Flüssigkeit oder eines Gels, die zur Verwendung als Handwaschzusammensetzung geeignet ist, umfassend Alkohol, Wasser und ein Verdickungsmittel, wobei die viskose Flüssigkeit oder das viskose Gel darin suspendierte Partikel aufweist, wobei die Partikel der Zusammensetzung eine körnige Textur verleihen und beim Reiben pflegende Substanzen freisetzen und verschwinden.

Bei einer Hautdesinfektion stellt sich das Problem, dass Desinfektionsmittel immer eine bestimmte Zeitspanne benötigen, bis sie ihre Wirkung entfaltet haben. Im Falle der Händedesinfektionsmittel auf Alkoholbasis beträgt diese Zeitspanne etwa 30 bis 60 Sekunden. Bisher wird dem Verwender in Form einer von diesem wahrzunehmenden Gebrauchsanleitung empfohlen, eine bestimmte Menge, in der Regel sind dies ca. 3ml, auf die Hand zu geben und gründlich zu verreiben. Doch auch bei einer solchen Empfehlung einer Anwendungszeit lässt sich die Zeitspanne für den Verwender nicht einfach überprüfen, es müsste ein Zeitmesser hinzugezogen werden.

JP H1 1-35403 A offenbart ein bakterizides Desinfektionsmittel, das ein antibakterielles Mittel, eine flüchtige Komponente und ein Pigment als Farbindikator enthält. Das Desinfektionsmittel wird in einen Gegenstand eingerieben. Während des Vorgangs kann der anhaftende Zustand des Desinfektionsmittels durch die Farbe des Farbstoffs identifiziert werden, während sich nach Ablauf der für die Desinfektion und Sterilisation des Gegenstands erforderlichen Zeit die flüchtige Komponente verflüchtigt und die Farbe des Farbstoffs kaum noch zu erkennen ist.

Es besteht daher ein Bedarf, eine Desinfektionszusammensetzung zur Verfügung zu stellen, die eine vorgegebene Anwendungszeit dem Verwender vermittelt. Diese Kenntnis würde die Effizienz der Verwendung von Desinfektionsmitteln steigern.

Aufgabe der vorliegenden Erfindung ist es daher, eine Verwendung einer Desinfektionszusammensetzung zur Hautdesinfektion zur Verfügung zu stellen, mit welcher eine Zeitspanne der Verwendung vermittelbar beziehungsweise bestimmbar ist.

Diese Aufgabe wird erfindungsgemäß gelöst durch eine nicht-therapeutische Verwendung einer Desinfektionszusammensetzung zur Hautdesinfektion in Form eines Gels, umfassend mindestens einen Alkohol, Wasser und mindestens ein Verdickungsmittel, wobei diese weiterhin wasserunlösliche Partikel umfasst, die am Ende eine Anwendungszeit abfallen, ausgewählt aus einer Gruppe umfassend Wachse, Polymere und/oder anorganische Stoffe, wobei die Partikel als Indikator für die vorgegebene Anwendungszeit dienen, wobei eine Größe der Partikel in einem Bereich von etwa 100 µm und etwa 1 mm liegt und wobei die wasserunlöslichen Partikel in einer Menge in einem Bereich von etwa 0,15 Gew.-% bis etwa 1,00 Gew.-%, bezogen auf die Gesamtmenge der Zusammensetzung, vorliegen.

Ein Gel ist ein disperses System, das aus mindestens einer festen Komponente, die ein Netzwerk bildet, und einer flüssigen Komponente, die in der festen Komponente immobilisiert ist, besteht. Die Viskosität der Desinfektionszusammensetzung liegt bevorzugt in einem Bereich von etwa 5 mPa*s bis etwa 50 mPa*s, weiter bevorzugt in einem Bereich von etwa 15 mPa*s bis etwa 30 mPa*s, gemessen mit einem Haake Viskotester bei 22,5 °C. Die Dichte der Desinfektionsmittelzusammensetzung liegt bevorzugt in einem Bereich von etwa 0,60 g/ml bis etwa 0,99 g/ml, weiter bevorzugt in einem Bereich von etwa 0,80 g/ml bis etwa 0,95 g/ml. Der pH-Wert der Desinfektionsmittelzusammensetzung liegt bevorzugt in einem Bereich von etwa 6,5 bis etwa 8,5, weiter bevorzugt in einem Bereich von etwa 7,0 bis etwa 8,0. Bei den angegebenen Werten zeigt die Desinfektionsmittelzusammensetzung ein für den Verwender angenehmes Fließverhalten bei Gebrauch bei hautschonenden Eigenschaften.

Wird im Rahmen der Erfindung der Begriff "etwa" im Zusammenhang mit Werten oder Wertebereichen verwendet, so ist darunter ein Toleranzbereich zu verstehen, den der Fachmann auf diesem Gebiet für üblich erachtet. Insbesondere ist ein Toleranzbereich von ±20 %, bevorzugt von ±10 % und weiter bevorzugt von ±5 % vorgesehen. Soweit verschiedene Bereiche für Mengenangaben in Bezug auf Bestandteile und für Definitionen, zum Beispiel von physikalisch-chemischen Eigenschaften der Zusammensetzung, in der vorliegenden Erfindung angegeben sind, sind die Untergrenzen und die Obergrenzen der verschiedenen Bereiche in Bezug auf den jeweiligen Bestandteil und auf die jeweilige Definition miteinander kombinierbar.

Erfindungsgemäß umfasst die Desinfektionszusammensetzung mindestens ein Verdickungsmittel. Ein Verdickungsmittel erhöht die Viskosität einer Lösung. Das mindestens eine Verdickungsmittel ist vorzugweise ausgewählt aus einer Gruppe umfassend Agaragar, Guar-Gum, Alginate, Xanthan-Gum, Gummiarabicum, Johannesbrotkernmehl, Leinsamengummen, Dextrane, Cellulosederivate, z.B. Methylcellulose, Hydroxyalkylcellulose und Carboxymethylcellulose, Stärkefraktionen und Derivate, wie Amylose, Amylopektin, Dextrine vernetzte und Poly(Acrylsäure). Weiter bevorzugt einsetzbar sind Polyvinylalkohol sowie teilweise verseifte Polyvinylacetate und deren Derivate. Die Erhöhung der Viskosität durch das Verdickungsmittel führt dazu, dass die Desinfektionszusammensetzung auf den Händen verbleibt und nicht leicht abtropft.

Bevorzugt umfasst die Desinfektionszusammensetzung Partikel, die wasser- und alkoholunlöslich sind. Die Partikel fallen bei der Benutzung der Desinfektionsmittelzusammensetzung durch den Verwender von den Händen oder dem Gesicht oder sonst zu reinigendem Körperteil ab, wenn der mindestens eine Alkohol der Zusammensetzung sich verflüchtigt hat. Hierdurch entfällt für den Verwender der durch die Partikel erzeugte leichte Reibeffekt, wodurch er die Rückmeldung erhält, dass die notwendige Anwendungszeit erreicht ist, um eine desinfizierende Wirkung zu erzielen.

Erfindungsgemäß dienen die Partikel als Indikator für eine vorgegebene Anwendungszeit. Die Anwendungszeit ist die Zeit, während welcher der Verwender sich die Hände oder ein sonstiges Körperteil mit dem Desinfektionsmittel reibt. Am Ende der Anwendungszeit ist kein Reibeffekt durch die Partikel mehr vorhanden, d.h. die Partikel reiben auf den Händen nicht mehr und fallen ab, da diese wasser- und alkoholunlöslich sind. Nur mit einer ausreichenden Anwendungszeit wird eine hinreichende Desinfektionswirkung erzielt. Vorzugsweise liegt die Anwendungszeit in einem Bereich von etwa 10 Sekunden bis etwa 180 Sekunden, weiter bevorzugt in einem Bereich von etwa 20 Sekunden bis etwa 120 Minuten, besonders bevorzugt in einem Bereich von etwa 30 Sekunden bis etwa 60 Sekunden.

Bevorzugt sind die Partikel farbig. Die Partikel sind so farblich im Desinfektionsmittel erkennbar, insbesondere wenn das Desinfektionsmittel sich auf der Haut des zu reinigenden Körperteils, insbesondere auf den Händen, befindet, und geben so dem Verwender auch eine optische Rückmeldung über ihr Vorhandensein neben dem Reibeffekt. Die Partikel sind bevorzugt nicht hautfarbig oder durchsichtig ausgebildet. Die Partikel können im Sinne der vorliegenden Erfindung zum Beispiel blau, grün oder rot sein, sie können aber auch weiß, grau oder schwarz sein, jedoch nicht hautfarbig. Auch Kombinationen verschiedenfarbiger Partikel sind möglich. Die Farbe der Partikel hat den Vorteil, dass für den Verwender die Prüfung erleichtert ist, ob diese von den Händen oder dem gereinigtem Körperteil abgefallen sind, oder ob sie noch vorhanden sind. Dies ist bei einer hautfarbenen Ausbildung der Partikel nicht gut möglich. Die Farbe gibt auch einen optischen Effekt gerade für junge Verwender wie Kinder. Die sonstigen Bestandteile der Desinfektionsmittelzusammensetzung sind farblos oder allenfalls leicht gefärbt.

Erfindungsgemäß liegt die Partikelgröße in einem Bereich von etwa 100 µm und etwa 1 mm, und bevorzugt in einem Bereich von etwa 400 µm und etwa 900 µm. Bevorzugt sind die Partikel sphärisch. Die sphärischen Partikel rollen sanft über die Haut und verursachen keine Hautrötungen.

Erfindungsgemäß umfasst die Desinfektionszusammensetzung die wasserunlöslichen Partikel in einer Menge in einem Bereich von etwa 0,15 Gew.-% bis etwa 1,00 Gew.-%, bezogen auf die Gesamtmenge der Zusammensetzung. Die Partikel sind vorzugweise ausgewählt aus einer Gruppe umfassend Paraffinwachse, Wachse, Polymere, und anorganische Stoffe. Wachse sind Ester von Ethylenglykol mit zwei Fettsäuren oder ein Monoester von Fettsäure und langkettigem Alkohol. Wachs ist bei 20 °C knetbar, fest bis brüchig-hart, weist eine feinkristalline Struktur auf, ist farblich durchscheinend bis opak, aber nicht glasartig. Wachs schmilzt über etwa 40°C ohne Zersetzung, ist oberhalb des Schmelzpunktes leicht flüssig (wenig viskos), weist eine stark temperaturabhängige Konsistenz und Löslichkeit auf und ist unter leichtem Druck polierbar. Ein Polymer im Sinne der vorliegenden Erfindung ist ein chemischer Stoff, der aus in Wasser unlöslichen Makromolekülen besteht. Die Makromoleküle eines Stoffes sind aus einer oder mehreren Struktureinheiten, den sogenannten konstitutionellen Repetiereinheiten oder Wiederholeinheiten, aufgebaut. Organische Stoffe sind Verbindungen, die Kohlenstoffatome enthalten. Bevorzugt ist der mindestens eine Alkohol ausgewählt aus einer Gruppe umfassend Ethanol, Isopropanol und Mischungen dieser. Alkohol hat den Vorteil einer kurzen Trocknungszeit. Die genannten Alkohole sind volatil. Bevorzugt liegt der mindestens eine Alkohol in der Zusammensetzung in einer Menge in einem Bereich von etwa 20 Gew.-% bis etwa 90 Gew.-%, weiter bevorzugt in einer Menge in einem Bereich von etwa 35 Gew.-% bis etwa 80 Gew.-%, noch weiter bevorzugt in einer Menge in einem Bereich von etwa 50 Gew.-% bis etwa 65 Gew.-%, bezogen auf die Gesamtmenge der Zusammensetzung, vor. Der mindestens eine Alkohol hat vorzugweise die Funktion eines Lösungsmittels als auch eines Desinfektionsmittels. Bevorzugt ist der mindestens eine Alkohol bakterizid, fungizid und/oder zumindest teilweise viruzid, und damit desinfizierend.

Bevorzugt liegt das Verhältnis zwischen der Menge an Partikel und der Menge an Alkohol in einem Bereich von etwa 0,01 % bis etwa 1 %, weiter bevorzugt in einem Bereich von etwa 0,05 % bis etwa 0,7 %, und noch weiter bevorzugt in einem Bereich von etwa 0,1 % bis etwa 0,5 % . Das Verhältnis zwischen der Menge an Partikel und der Menge an Alkohol ist auf die Anwendungsdauer des Zusammensetzung abgestimmt, bei der also der Reibeffekt fortfällt und damit die ausreichende Anwendungsdauer dem Verwender rückgemeldet wird.

Das Wasser in der Desinfektionsmittelzusammensetzung hat die Funktion eines Lösungsmittels, jedoch nicht für die wasserunlöslichen Partikel. Das Wasser kann vorzugsweise Leistungswasser oder deionisiertes Wasser sein. Deionisiertes Wasser ist Wasser ohne die im Quell- und Leitungswasser vorkommenden Salze, die als Anionen und Kationen gelöst sind, und wird in der Chemie und der Biologie als Lösungs- und manchmal auch als Reinigungsmittel verwendet. Das Wasser hat den Vorteil, eine längere Trocknungszeit als Alkohol aufzuweisen. Des Weiteren ist eine gewisse Wassermenge auch für eine bestmögliche Desinfektionsleistung notwendig. Reine Alkohole desinfizieren schlechter als solche, die mit etwa 10 Gew.-% bis etwa 40 Gew.-% Wasser, bezogen auf die Gesamtmenge der Mischung aus Wasser und Alkohol, verdünnt werden. Der in der Desinfektionsmittelzusammensetzung vorliegende mindestens eine Alkohol weist bevorzugt eine kürzere Trocknungszeit auf als das Wasser. Das Wasser liegt vorzugweise in der Zusammensetzung in einer Menge in einem Bereich von etwa 10 Gew.-% bis etwa 80 Gew.-%, weiter bevorzugt in einer Menge in einem Bereich von etwa 20 Gew.-% bis etwa 65 Gew.-%, noch weiter bevorzugt in einer Menge in einem Bereich von etwa 30 Gew.-% bis etwa 50 Gew.-%, bezogen auf die Gesamtmenge der Zusammensetzung, vor.

Bevorzugt liegt das Verhältnis zwischen der Menge an Wasser und der Menge an Alkohol beziehungsweise Alkoholen in einem Bereich von etwa 0,3 bis etwa 1, weiter bevorzugt in einem Bereich von etwa 0,4 bis etwa 0,9, und noch weiter bevorzugt in einem Bereich von etwa 0,5 bis etwa 0,8. Das Verhältnis zwischen der Menge an Wasser und der Menge an Alkohol ist auf die Anwendungsdauer des Reibeffektes abgestimmt.

Bevorzugt kann mindestens eine Alkalie, auch Alkalimittel genannt, in der Zusammensetzung vorliegen. Alkalien sind wasserlösliche Laugen. Die mindestens eine Alkalie liegt bevorzugt in einer Menge in einem Bereich von etwa 0,10 Gew.-% bis etwa 0,80 Gew.-%, weiter bevorzugt in einer Menge in einem Bereich von etwa 0,20 Gew.-% bis etwa 0,65 Gew.-%, noch weiter bevorzugt in einer Menge in einem Bereich von etwa 0,25 Gew.-% bis etwa 0,50 Gew.-%, bezogen auf die Gesamtmenge der Zusammensetzung, in dieser vor. Die Alkalie ist vorzugweise ausgewählt aus einer Gruppe umfassend Aminomethylpropanol, Diisopropanolamine, Triethanolamine und weitere Amine aus dem kosmetischen Bereich. Mittels der mindestens einen Alkalie kann der pH-Wert der Desinfektionszusammensetzung geändert beziehungsweise eingestellt werden. Gesunde Haut hat einen leicht sauren pH-Wert und erreicht maximal pH-Werte von etwa 6 bis 6,5. Ein sehr basischer oder sehr saurer pH-Wert kann zu Hautproblemen oder gar Hautkrankheiten führen. Mittel, die im Kontakt mit Haut sind, müssen daher einen pH-Wert aufweisen, der das Gleichgewicht des Hydrolipidfilms nicht beeinträchtigt.

Bevorzugt kann mindestens ein Mittel zur Verbesserung der Hautverträglichkeit in der Zusammensetzung vorliegen. Mittel zur Verbesserung der Hautverträglichkeit umfassen Substanzen, die sicherstellen, dass die Haut beim Auftragen des Gels nicht gereizt oder sonst wie in Mitleidenschaft gezogen wird. Diese Mittel schützen damit die Haut. Bevorzugt kann mindestens ein Feuchthaltemittel als Mittel zur Verbesserung der Hautverträglichkeit in der Zusammensetzung vorliegen. Feuchthaltemittel sind Zusatzstoffe, die das Austrocknen verhindern, indem sie bei der Herstellung der Desinfektionsmittelzusammensetzung zugesetztes Wasser binden oder bei der Lagerung beziehungsweise nach Benutzungsaufnahme der Desinfektionsmittelzusammensetzung durch den Verwender Luftfeuchtigkeit an sich ziehen. Das mindestens ein Mittel zur Verbesserung der Hautverträglichkeit und/oder ein Feuchthaltemittel liegt in einer Menge in einem Bereich von etwa 0,02 Gew.-% bis etwa 10,00 Gew.-%, weiter bevorzugt in einer Menge in einem Bereich von etwa 0,10 Gew.-% bis etwa 4,00 Gew.-%, und noch weiter bevorzugt in einer Menge in einem Bereich von etwa 0,50 Gew.-% bis etwa 2,00 Gew.-%, bezogen auf die Gesamtmenge der Zusammensetzung, in dieser vor. Das mindestens eine Mittel zur Verbesserung der Hautverträglichkeit ist vorzugweise ausgewählt aus einer Gruppe umfassend Aloe Vera Gel/Saft, Glycerin, Propan-1,2-diol, Butan-1,3-diol, Sorbitol, Dexpanthenol, Allantoin, Bisabolol, Tocoferylacetat, Dodecanol, Tetradecanol, Hexadecanol, Octadecanol, Lanolinalkohol, Cetearylalkohol, Cetylstearylalkohol Cyclomethicone, Dimethicone, Polydimethylsiloxan, Isopropylmyristat, Isopropylpalmitat, Cetearlethylhexa-noat, Octylstearat, Octyloctonoat, Ethylhexansäure. Ethylester, Jojobaöl, Sanddornöl, Wollwachs, Parafinöl, Vaselin, Heptamethylnonan/Isohexandecan, Cholesterol, Partialglyceriden und -triglyceriden, Glycerin, Dicaprylylether, Dioctylether, Diisodecyladipat, Adipinsäurediisodecylester, Hyaluronsäure, Natriumlactat, und Harnstoff.

Weiter bevorzugt kann mindestens ein Rückfettungsmittel als Mittel zur Verbesserung der Hautverträglichkeit in der Zusammensetzung vorliegen. Rückfettungsmittel sind lipophile Grundstoffe, die eine störende Auswirkung auf die epidermale Barrierefunktion verhindern sollen. Das mindestens eine Rückfettungsmittel liegt bevorzugt in einer Menge in einem Bereich von etwa 0,01 Gew.-% bis etwa 5,00 Gew.-%, weiter bevorzugt in einer Menge in einem Bereich von etwa 0,10 Gew.-% bis etwa 4,00 Gew.-%, und noch weiter bevorzugt in einer Menge in einem Bereich von etwa 0,50 Gew.-% bis etwa 2,00 Gew.-%, bezogen auf die Gesamtmenge der Zusammensetzung, in dieser vor. Das mindestens eine Rückfettungsmittel ist vorzugweise ausgewählt aus einer Gruppe umfassend Polyglykoether, Glycerintri-, Glycerindi- und Glycerinmonooleat, Glycerylcaprylat, Glycerylcaprat, Polyglyceryl-2-caprat, Glycerinstearat, glycerinmonostearat, Stearinsäure-Glycerinestesr, Isopropylmyristat, Myristinsäureisopropylester, Isopropylpalmitaat, Palmitinsäure-Isopropylester, Macadamianussöl, 2-Octyldodecan-1-ol, Hartparaffin, mikrokristallines Wachs, Ozokerit, Ceresin, Vaselin (auf Mineralölbasis), flüssiges Paraffin, Paraffinöl (auf Mineralölbasis), Avocadoöl, Jojobaöl, Stearinsäure, Octadecansäure, n-Octodecansäure sowie langkettigen linearen oder verzweigten ein- oder mehrwertigen Fettolkoholen, beispielsweise Octyldodecanol. Gleichermaßen ist Isopropylmyristat und Cetearyloctonoat eine rückfettende Wirkung zugeordnet. Besonders bevorzugt können mindestens ein Rückfettungsmittel und mindestens ein Feuchthaltemittel als Mittel zur Verbesserung der Hautverträglichkeit in der Zusammensetzung vorliegen.

Bevorzugt kann mindestens ein Parfumöl von der Zusammensetzung umfasst sein, bevorzugt in einer Menge in einem Bereich von etwa 0,01 Gew.-% bis etwa 2,00 Gew.-%, weiter bevorzugt in einer Menge in einem Bereich von etwa 0,05 Gew.-% bis etwa 1,00 Gew.-%, und noch weiter bevorzugt in einer Menge in einem Bereich von etwa 0,10 Gew.-% bis etwa 0,50 Gew.-%, bezogen auf die Gesamtmenge der Zusammensetzung. Der Vorteil der Verwendung von mindestens einem Parfümöl ist, dass die Anwendung der Desinfektionszusammensetzung angenehmer wird, da Alkohol einen starken Geruch hat.

Eine beispielhafte Desinfektionszusammensetzung umfasst bevorzugt mindestens einen Alkohol in einer Menge in einem Bereich von etwa 20 Gew.-% bis etwa 90 Gew.-%, Wasser in einer Menge in einem Bereich von etwa 10 Gew.-% bis etwa 80 Gew.-%, mindestens ein Verdickungsmittel in einer Menge in einem Bereich von etwa 0,01 Gew.-% bis etwa 2,00 Gew.-%, und Partikel, die wasserunlöslich sind, ausgewählt aus einer Gruppe umfassend Wachse, Polymere und/oder anorganische Stoffe, in einer Menge in einem Bereich von etwa 0,15 Gew.-% bis etwa 1,00 Gew.-%, wobei die Gewichtsprozentangaben jeweils bezogen auf die Gesamtmenge der Zusammensetzung.

Eine beispielhafte Desinfektionszusammensetzung umfasst weiter bevorzugt mindestens einen Alkohol in einer Menge in einem Bereich von etwa 35 Gew.-% bis etwa 80 Gew.-%, Wasser in einer Menge in einem Bereich von etwa 20 Gew.-% bis etwa 65 Gew.-%, mindestens ein Verdickungsmittel in einer Menge in einem Bereich von etwa 0,10 Gew.-% bis etwa 1,50 Gew.-%, und Partikel, die wasserunlöslich sind, ausgewählt aus einer Gruppe umfassend Wachse, Polymere und/oder anorganische Stoffe, in einer Menge in einem Bereich von etwa 0,15 Gew.-% bis etwa 1,00 Gew.-%, wobei die Gewichtsprozentangaben jeweils bezogen auf die Gesamtmenge der Zusammensetzung.

Eine beispielhafte Desinfektionszusammensetzung umfasst noch weiter bevorzugt mindestens einen Alkohol in einer Menge in einem Bereich von etwa 50 Gew.-% bis etwa 65 Gew.-%, Wasser in einer Menge in einem Bereich von etwa 30 Gew.-% bis etwa 50 Gew.-%, mindestens ein Verdickungsmittel in einer Menge in einem Bereich von etwa 0,15 Gew.-% bis etwa 1,00 Gew.-%, und Partikel, die wasserunlöslich sind, ausgewählt aus einer Gruppe umfassend Wachse, Polymere und/oder anorganische Stoffe, in einer Menge in einem Bereich von etwa 0,15 Gew.-% bis etwa 1,00 Gew.-%, wobei die Gewichtsprozentangaben jeweils bezogen auf die Gesamtmenge der Zusammensetzung.

Eine beispielhafte Desinfektionszusammensetzung umfasst noch weiter bevorzugt Ethanol in einer Menge in einem Bereich von etwa 20 Gew.-% bis etwa 90 Gew.-%, Wasser in einer Menge in einem Bereich von etwa 10 Gew.-% bis etwa 80 Gew.-%, Polyacrylsäure als Verdickungsmittel in einer Menge in einem Bereich von etwa 0,01 Gew.-% bis etwa 2,00 Gew.-%, und mindestens ein Paraffinwachs, das wasserunlöslich ist, als Partikel in einer Menge in einem Bereich von etwa 0,15 Gew.-% bis etwa 1,00 Gew.-%, wobei die Gewichtsprozentangaben jeweils bezogen auf die Gesamtmenge der Zusammensetzung.

Eine beispielhafte Desinfektionszusammensetzung umfasst noch weiter bevorzugt Propanol einer Menge in einem Bereich von etwa 20 Gew.-% bis etwa 90 Gew.-%, Wasser in einer Menge in einem Bereich von etwa 10 Gew.-% bis etwa 80 Gew.-%, Polyacrylsäure als Verdickungsmittel in einer Menge in einem Bereich von etwa 0,01 Gew.-% bis etwa 2,00 Gew.-%, und mindestens ein Paraffinwachs, das wasserunlöslich ist, als Partikel in einer Menge in einem Bereich von etwa 0,15 Gew.-% bis etwa 1,00 Gew.-%, wobei die Gewichtsprozentangaben jeweils bezogen auf die Gesamtmenge der Zusammensetzung.

Eine beispielhafte Desinfektionszusammensetzung umfasst noch weiter bevorzugt eine Mischung von Ethanol und Propanol in einer Menge in einem Bereich von etwa 20 Gew.-% bis etwa 90 Gew.-%, wobei die Menge an Ethanol bevorzugt in einem Bereich von etwa 13 Gew.-% und etwa 60 Gew.-% und die Menge an Propanol bevorzugt in einem Bereich von etwa 7 Gew.-% bis etwa 30 Gew.-% liegt, Wasser in einer Menge in einem Bereich von etwa 10 Gew.-% bis etwa 80 Gew.-%, Polyacrylsäure als Verdickungsmittel in einer Menge in einem Bereich von etwa 0,01 Gew.-% bis etwa 2,00 Gew.-%, und mindestens ein Paraffinwachs, das wasserunlöslich ist, als Partikel in einer Menge in einem Bereich von etwa 0,15 Gew.-% bis etwa 1,00 Gew.-%, wobei die Gewichtsprozentangaben jeweils bezogen auf die Gesamtmenge der Zusammensetzung.

Eine beispielhafte Desinfektionszusammensetzung umfasst noch weiter bevorzugt Ethanol in einer Menge in einem Bereich von etwa 20 Gew.-% bis etwa 90 Gew.-%, Wasser in einer Menge in einem Bereich von etwa 10 Gew.-% bis etwa 80 Gew.-%, Polyacrylsäure als Verdickungsmittel in einer Menge in einem Bereich von etwa 0,01 Gew.-% bis etwa 2,00 Gew.-%, und mindestens ein Polymer, das wasserunlöslich ist, als Partikel in einer Menge in einem Bereich von etwa 0,15 Gew.-% bis etwa 1,00 Gew.-%, wobei die Gewichtsprozentangaben jeweils bezogen auf die Gesamtmenge der Zusammensetzung.

Eine beispielhafte Desinfektionszusammensetzung umfasst noch weiter bevorzugt Propanol in einer Menge in einem Bereich von etwa 20 Gew.-% bis etwa 90 Gew.-%, Wasser in einer Menge in einem Bereich von etwa 10 Gew.-% bis etwa 80 Gew.-%, Polyacrylsäure als Verdickungsmittel in einer Menge in einem Bereich von etwa 0,01 Gew.-% bis etwa 2,00 Gew.-%, und mindestens ein Polymer, das wasserunlöslich ist, als Partikel in einer Menge in einem Bereich von etwa 0,15 Gew.-% bis etwa 1,00 Gew.-%, wobei die Gewichtsprozentangaben jeweils bezogen auf die Gesamtmenge der Zusammensetzung.

Eine beispielhafte Desinfektionszusammensetzung umfasst noch weiter bevorzugt eine Mischung von Ethanol und Propanol in einer Menge in einem Bereich von etwa 20 Gew.-% bis etwa 90 Gew.-%, wobei die Menge an Ethanol bevorzugt in einem Bereich von etwa 13 Gew.-% und etwa 60 Gew.-% und die Menge an Propanol bevorzugt in einem Bereich von etwa 7 Gew.-% bis etwa 30 Gew.-% liegt, Wasser in einer Menge in einem Bereich von etwa 10 Gew.-% bis etwa 80 Gew.-%, Polyacrylsäure als Verdickungsmittel in einer Menge in einem Bereich von etwa 0,01 Gew.-% bis etwa 2,00 Gew.-%, mindestens ein Polymer, das wasserunlöslich ist, als Partikel in einer Menge in einem Bereich von etwa 0,15 Gew.-% bis etwa 1,00 Gew.-%, wobei die Gewichtsprozentangaben jeweils bezogen auf die Gesamtmenge der Zusammensetzung.

Des Weiteren können die vorgenannten konkreten Zusammensetzungen umfassen:
- mindestens eine Alkalie in einer Menge in einem Bereich von etwa 0,1 Gew-% bis etwa 0,8 Gew-%,
- mindestens ein Feuchthaltemittel als Mittel zur Verbesserung der Hautverträglichkeit in einer Menge in einem Bereich von etwa 0,01 Gew-% bis etwa 12,00 Gew-%,mindestens ein Rückfettungsmittel als weiteres Mittel zur Verbesserung der Hautverträglichkeit in einer Menge in einem Bereich von etwa 0,01 Gew-% bis etwa 5,00 Gew-%, und/oder
- mindestens ein Parfumöl in einer Menge in einem Bereich von etwa 0,01 Gew-% bis etwa 5,00 Gew-%.

Die vorliegende Erfindung betrifft eine Verwendung einer Desinfektionszusammensetzung, bei der die Partikel als Indikator für eine vorgegebene Anwendungszeit dienen. Das Ende der Anwendung legt klar fest, wenn die Farbe und/oder der Reibeffekt nicht mehr vorhanden sind. Die Händedesinfektionsmittel auf Alkoholbasis beträgt diese Zeitspanne ca. 30-60 Sekunden. Desinfektionsmittel der vorliegenden Erfindung ist während einer bestimmten Zeitspanne verwendet, bis sie ihre Wirkung entfaltet haben.

Anhand des folgenden Ausführungsbeispiels wird die vorliegende Erfindung näher erläutert. Es wurde eine Desinfektionszusammensetzung hergestellt aus 40 Gew.-% Ethanol, 19 Gew.-% Propanol, 37,80 Gew.-% Wasser, 0,35 Gew.-% eines modifiziertes Acrylpolymers als Verdickungsmittel, 0,30 Gew.-% eines Alkali, 1,15 Gew.-% eines Feuchthaltemittels als Mittel zur Verbesserung der Hautverträglichkeit, 1,00 Gew.-% eines Rückfettungsmittel, 0,20 Gew.-% Parfumöl, und 0,20 Gew.-% wasserunlösliche Partikel, bestehend aus Paraffinwachs mit einer Partikelgröße in einem Bereich von 420 µm bis 840 µm. Die Gew.-%-Angaben beziehen sich dabei auf die Gesamtmenge der Desinfektionszusammenfassung. Der Verdickungsmittel wurde zunächst in Wasser gelöst und während mindestens einer Stunde zur Herstellung einer Vorlösung gerührt. Das Parfum wurde in Alkohol gelöst. Das Rückfettungsmittel und der Farbstoff wurden in der Mischung zugegeben. Dem Alkali und dem Mittel zur Verbesserung der Hautverträglichkeit wurde Wasser zugegeben. Zur Verdickungsmittel-Vorlösung wurde die Alkohol-Parfum-Lösung zugegeben. Die Lösung wurde 15 min gerührt. Die Rührgeschwindigkeit wurde minimiert und die Lösung wurde noch 30 min gerührt. Die Alkali-Lösung wurde langsam zugegeben und die Rührgeschwindigkeit langsam erhöht. Die Lösung wurde noch 10 min gerührt. Die Partikel, die wasserunlöslich sind, wurden als letzte Komponente hinzugegeben.

Mit dem Desinfektionsmittel und der erfindungsgemäßen Verwendung wird ein einfach für den Endverbraucher anzuwendendes Desinfektionsmittel mit Zeitindikator zur Verfügung gestellt.

## Patentansprüche

1. Nicht-therapeutische Verwendung einer Desinfektionszusammensetzung zur Hautdesinfektion, in der Form eines Gels, umfassend mindestens einen Alkohol, Wasser und mindestens ein Verdickungsmittel, **dadurch gekennzeichnet, dass** diese weiterhin wasserunlösliche Partikel umfasst, die am Ende einer Anwendungszeit abfallen, ausgewählt aus einer Gruppe umfassend Wachse, Polymere und/oder anorganische Stoffe, wobei die Partikel als Indikator für die vorgegebene Anwendungszeit dienen, wobei eine Größe der Partikel in einem Bereich von etwa 100 µm und etwa 1 mm liegt und wobei die wasserunlöslichen Partikel in einer Menge in einem Bereich von etwa 0,15 Gew.-% bis etwa 1,00 Gew.-%, bezogen auf die Gesamtmenge der Zusammensetzung, vorliegen.

2. Verwendung nach Anspruch 1, **dadurch gekennzeichnet, dass** die Partikel farbig sind.

3. Verwendung nach einem oder mehreren der vorhergehenden Ansprüche, **dadurch gekennzeichnet, dass** der mindestens eine Alkohol ausgewählt ist aus einer Gruppe umfassend Ethanol, Isopropanol und Mischung dieser.

4. Verwendung nach einem oder mehreren der vorhergehenden Ansprüche, **dadurch gekennzeichnet, dass** der mindestens eine Alkohol in der Zusammensetzung in einer Menge in einem Bereich von etwa 20 Gew.-% bis etwa 90 Gew.-%, bezogen auf die Gesamtmenge der Zusammensetzung, vorliegt.

5. Verwendung nach einem oder mehreren der vorhergehenden Ansprüche, **dadurch gekennzeichnet, dass** das Wasser in der Zusammensetzung in einer Menge in einem Bereich von etwa 10 Gew.-% bis etwa 80 Gew.-%, bezogen auf die Gesamtmenge der Zusammensetzung, vorliegt.

## Claims

1. Non-therapeutic use of a disinfectant composition for skin disinfection, in the form of a gel comprising at least one alcohol, water and at least one thickening agent, **characterized in that** it further comprises water-insoluble particles which fall off at the end of an application time, selected from a group comprising waxes, polymers and/or inorganic substances, wherein the particles serve as an indicator for the predetermined application time, wherein a size of the particles is in a range of about 100 µm and about 1 mm and wherein the water-insoluble particles are present in an amount in a range of about 0.15 wt.-% to about 1.00 wt.-%, based on the total amount of the composition.

2. Use according to claim 1, **characterized in that** the particles are coloured.

3. Use according to one or more of the preceding claims, **characterized in that** the at least one alcohol is selected from a group comprising ethanol, isopropanol and mixtures thereof.

4. Use according to one or more of the preceding claims, **characterized in that** the at least one alcohol is present in the composition in an amount in a range from about 20 wt.-% to about 90 wt.-%, based on the total amount of the composition.

5. Use according to one or more of the preceding claims, **characterized in that** the water is present in the composition in an amount ranging from about 10 wt.-% to about 80 wt.-%, based on the total amount of the composition.

## Revendications

1. Utilisation non thérapeutique d'une composition désinfectante pour désinfecter la peau, se présentant sous forme de gel, et comprenant au moins un alcool, de l'eau et au moins un agent épaississant, **caractérisée en ce qu'**elle comprend en outre des particules insolubles dans l'eau qui se détachent à la fin d'un temps d'application et qui sont sélectionnées dans un groupe comprenant des cires, des polymères et/ou des substances inorganiques, dans laquelle les particules servent d'indicateur pour le temps d'application prédéterminé, dans laquelle la taille des particules est comprise à l'intérieur d'une plage allant d'environ 100 µm à environ 1 mm, et dans laquelle les particules insolubles dans l'eau sont présentes en une quantité qui est comprise à l'intérieur d'une plage allant d'environ 0,15 % en poids à 1,00 % en poids, sur la base de la quantité totale de la composition.

2. Utilisation selon la revendication 1, **caractérisée en ce que** les particules sont colorées.

3. Utilisation selon une ou plusieurs des revendications précédentes, **caractérisée en ce que** ledit au moins un alcool est sélectionné dans un groupe comprenant l'éthanol, l'isopropanol et un mélange de ceux-ci.

4. Utilisation selon une ou plusieurs des revendications précédentes, **caractérisée en ce que** ledit au moins un alcool est présent dans la composition en une quantité qui est comprise à l'intérieur d'une plage allant d'environ 20 % en poids à environ 90 % en poids, sur la base de la quantité totale de la composition.

5. Utilisation selon une ou plusieurs des revendications précédentes, **caractérisée en ce que** l'eau est présente dans la composition en une quantité qui est comprise à l'intérieur d'une plage allant d'environ 10 % en poids à environ 80 % en poids, sur la base de la quantité totale de la composition.
